# EUROPEAN PATENT APPLICATION

(11) **EP 3 670 642 A1**
(43) Date of publication of application: **24.06.2020**
(21) Application number: 18845670.1
(22) Date of filing: 14.08.2018
(51) Int. Cl.: C12M 1/00, C12N 1/12

(54) **MICROORGANISM CULTURE SYSTEM**

(30) Priority: 16.08.2017 JP 2017157237
(71) Applicant: Nippon Soda Co., Ltd., Tokyo 100-8165 (JP)
(72) Inventor: IWAKOSHI Banri, Tokyo 100-8165 (JP); YONEYA Akira, Katori-shi Chiba 289-0405 (JP)
(74) Representative: Wibbelmann, Jobst
(86) International application number: PCT/JP2018/030280
(87) International publication number: WO 2019/035455

(57) **Abstract**

The microorganism culture system (1) includes: flat carriers (2) to which microorganisms are to be attached; a culture solution supply unit (3) that supplies a culture solution to the carriers (2) from above the carriers (2); and an effluent tank (5) that stores a culture solution containing the microorganisms flowing out of the carriers (2). The plurality of carriers (2) are arranged so that surfaces of the carriers (2) are directly opposed to each other or obliquely face each other at an angle. Light irradiation units (4) are installed between the carriers (2) and in at least a part of an outside of the carriers (2) in a horizontal direction and an outside thereof in a vertical direction when viewed in an arrangement direction of the carriers (2).

## Description

### Technical Field

The present invention relates to a microorganism culture system.

Priority is claimed on Japanese Patent Application No. 2017-157237, filed August 16, 2017, the content of which is incorporated herein by reference.

### Background Art

Attempts to suppress emission of greenhouse gas as much as possible and the like have been strongly required in industries in various countries as a measure against global warming. Microorganisms such as photosynthetic bacteria or microalgae such as *Chlorella* are extremely promising as resources enabling production of energy without emitting CO₂ and are expected to be utilized on a commercial level and to be efficiently produced.

It is required to produce microalgae such as *Chlorella* at as low a cost as possible in order to use them for energy resources or in other industrial uses. However, large pools or tanks are required in the case of mass-culturing microalgae in water. Accordingly, there are problems such as an increase in cost due to acquisition of land or large facilities.

In order to improve the production amount per unit area using simple facilities and effectively utilizing land, Patent Literature 1 proposes a culture system in which a culture solution is allowed to naturally flow down on the surface of a vertically standing carrier, microorganisms such as microalgae are made to continuously proliferate on the surface of the carrier, and the microorganisms are continuously collected from the culture solution which has been allowed to naturally flow down. In this system, a thin water film on the surface of the carrier corresponds to the water surface of a pool of a method in the related art, and photosynthesis is performed by obtaining light (artificial light), carbonic acid gas, and nutrients. In a unit in which this system is stored, a culture volume the same as or larger than the culture volume on the water surface having the same area as that of a sheet of a carrier can be obtained, and a harvest of 10 to 20 times as many microorganisms as in a method in the related art using a pool or the like can be expected from the same floor area using parallel multilayer equipment of carriers.

Furthermore, a culture volume 100 times that of a method in the related art per floor area can be expected to be secured by stacking the units vertically. According to such a culture system, it is possible to overcome location constraints that limit use of the methods to sunlight-rich areas and to perform culture in a polar region or a basement, or even in outer space.

However, the culture system disclosed in Patent Literature 1 has problems in that the efficiency of transmitting light energy is poor and the efficiency of culturing microorganisms is low.

A device in which a plurality of plate-like carriers and plate-like light sources are installed parallel to each other and which cultures photosynthetic microorganisms has been proposed in Patent Literature 2 to solve such problems. In this device, the carriers and the light sources are alternately arranged in a gas phase, and a culture solution is supplied to each carrier from above each carrier.

### [Citation List]

### [Patent Literature]

[Patent Literature 1] Japanese Unexamined Patent Application, First Publication No. 2013-153744
[Patent Literature 2] Japanese Unexamined Patent Application, First Publication No. H06-23389

### SUMMARY OF INVENTION

### Technical Problem

However, the device disclosed in Patent Literature 2 has a configuration in which the plate-like light sources are alternately arranged between the plate-like carriers. Therefore, the interval between unit culturing devices (also referred to as "cells") each having a carrier and a light source as a set is physically restricted, and there is a limit in increasing the mounting density even if production capacity per floor area is secured by arranging a plurality of cells in parallel.

In addition, there is a problem in that it is difficult to collect microorganisms from carriers because the light source disposed between the carriers becomes an obstacle. Furthermore, since a large light source facing almost the entire surface of a carrier is used, there is a problem in that the energy that is used becomes excessive.

The present invention provides a culture system in which carriers and light irradiation units are efficiently installed and microorganisms are easily collected from the carriers and can be efficiently produced using a light source with lower energy consumption.

### Solution to Problem

The present inventors have conducted extensive studies in order to solve the above-described problems, and as a result, have completed the invention having the following configuration.
(1) A microorganism culture system according to a first aspect of the present invention includes: flat carriers to which microorganisms are to be attached; a culture solution supply unit that supplies a culture solution from above the carriers; and an effluent tank that stores a culture solution containing the microorganisms flowing out of the carriers, in which the plurality of carriers are arranged so that surfaces of the carriers are directly opposed to each other or obliquely face each other at an angle, and light irradiation units are installed between the plurality of arranged carriers and on an outside of the carriers in a horizontal direction and/or an outside thereof in a vertical direction when viewed in an arrangement direction of the carriers. That is, culture surfaces of the adjacent carriers to which microorganisms are to be attached may be arranged to face each other in parallel, or may be arranged to form a certain angle with each other. The certain angle may be, for example, an angle of 0° or more and 120° or less. In the case where the culture surfaces are arranged at a certain angle, side edges of the adjacent carriers which are close to each other may be arranged in contact with each other or at constant intervals. In this case, the adjacent carriers may be arranged in substantially an L shape when viewed in plan view (seen from above), or may be arranged in a zigzag shape as a whole when viewed in plan view.
(2) The microorganism culture system according to (1), in which the light irradiation units are arranged so as to be directly opposed to the surfaces of the carriers.
(3) The microorganism culture system according to (1) or (2), in which a photon flux density on the surfaces of the carriers in a wavelength range in which the microorganisms can be absorbed is greater than or equal to 50 µmolm⁻²s⁻¹. The photon flux density is the number of photons passing through a unit area per unit time. In a case where there are no microorganisms specified, a photon flux density (photosynthesis-effective photon flux density: PPFD) at a wavelength of 400 nm to 700 nm which is generally effective for photosynthesis may be used as the above-described value, for example.
(4) The microorganism culture system according to any one of (1) to (3), in which the light irradiation units have a plurality of LED bulbs (or LEDs, the same applies hereinafter) arranged in a row and have a plurality of lenses which can perform adjustment so that an even amount of light is supplied to the entire surfaces of the carriers and each of which is disposed opposite to each of the LED bulbs.
(5) The microorganism culture system according to any one of (1) to (4), in which the light irradiation units are arranged at positions interposed between side edges opposed to each other on at least one of a left side or a right side when the carriers are viewed in the arrangement direction. Linear light irradiation units may be arranged between the side edges, which face each other, of the adjacent carriers substantially in parallel to the side edges. In a case where the adjacent carriers are arranged in substantially an L shape when viewed in plan view, the linear light irradiation units may be arranged at positions facing valley portions of the L shape substantially in parallel to the side edges.
(6) The microorganism culture system according to any one of (1) to (5), in which the microorganisms are microalgae.

### Advantageous Effects of Invention

The microorganism culture system of the present invention exhibits an effect of improving the efficiency of installing light sources and carriers with respect to a floor area. In addition, the microorganism culture system of the present invention exhibits effects that microorganisms are easily collected from the carriers and can be efficiently produced using light irradiation units with lower energy consumption.

### BRIEF DESCRIPTION OF THE DRAWINGS

FIG. 1 is a perspective view schematically showing a microorganism culture system according to an embodiment of the present invention.
FIG. 2 is a cross-sectional view taken along line A-A of FIG. 1 which is indicated by an arrow.
FIG. 3 is a perspective view schematically showing an arrangement state of carriers and light irradiation units of the microorganism culture system according to the embodiment of the present invention.
FIG. 4 is a perspective view schematically showing an arrangement state of carriers and light irradiation units of the microorganism culture system according to the embodiment of the present invention.
FIG. 5 is a plan view schematically showing a modification example of an arrangement state of carriers and light irradiation units of the microorganism culture system according to the embodiment of the present invention.

### DESCRIPTION OF EMBODIMENTS

Hereinafter, an embodiment of a microorganism culture system of the present invention will be described with reference to the drawings.

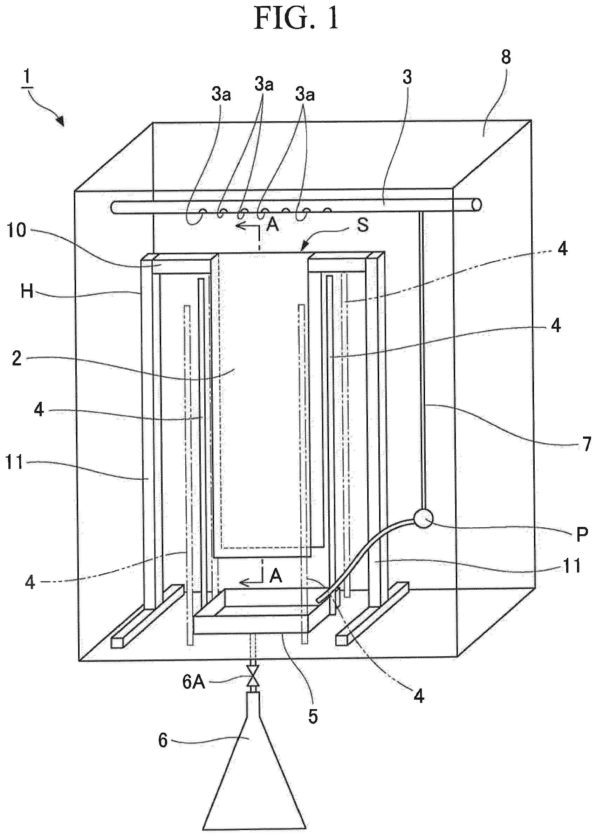

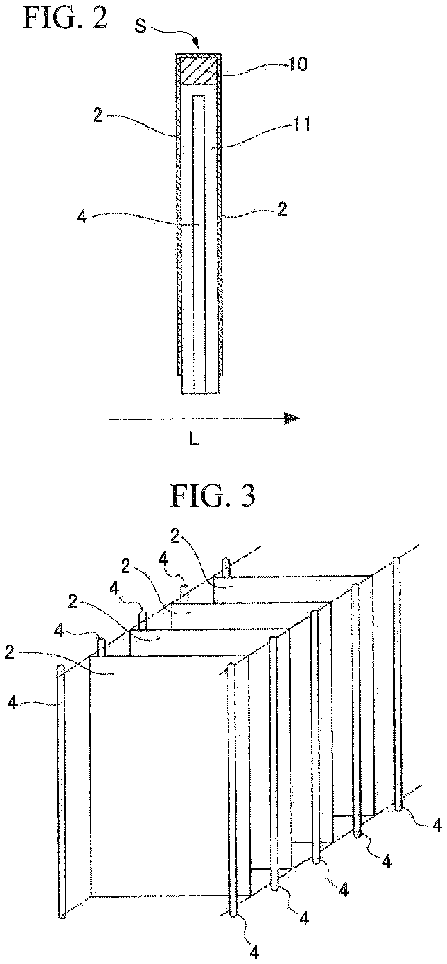

The culture system according to the present embodiment is a system for culturing microorganisms in a gas phase as schematically shown in FIG. 1 or 2 and includes carriers 2 arranged in a substantially vertical direction, a culture solution supply unit 3 that supplies a culture solution to the carriers 2, light irradiation units 4 that irradiate the carriers 2 with light, an effluent tank 5 that stores a culture solution containing microorganisms flowing out of the carriers 2, a harvest container 6 that accommodates microorganisms separated from the culture solution stored in the effluent tank 5, a circulation flow path 7 that circulates a culture solution separated from the culture solution stored in the effluent tank 5, and a case 8 that covers the carriers 2, the culture solution supply unit 3, the effluent tank 5, and the circulation flow path 7.

In this embodiment, a flexible rectangular sheet S is bent in an inverted U shape at the center in a longitudinal direction, a pair of rectangular portions hung parallel to each other form a pair of flat carriers 2, and an inside or outside surface of the sheet S becomes a culture surface. Microorganisms can be attached to the carriers 2, and a culture solution supplied from above can be allowed to flow down while being allowed to permeate into the carriers 2. Therefore, the water capacity of the carriers per unit area is preferably greater than or equal to 0.2 g/cm². The "water capacity" in the present specification means a value measured from a water retention test described in examples to be described below. The water capacity of the carriers 2 per unit area is more preferably greater than or equal to 0.25 g/cm² and still more preferably greater than or equal to 0.3 g/cm². The upper limit of the water capacity of the carriers 2 per unit area is not particularly limited, but can be selected from ranges of less than or equal to 10 g/cm², less than or equal to 8 g/cm², less than or equal to 5 g/cm², less than or equal to 3 g/cm², less than or equal to 1 g/cm², and the like.

Any material that can hold microorganisms and a culture solution may be used as the material of the carriers 2, and cloth, non-woven fabric, felt, a spongy material, and other porous materials can be used. Preferred specific examples thereof include pile fabrics of twisted yarn or non-twisted yarn. Pile fabrics of non-twisted yarn are particularly preferable. The material of pile fabrics is not particularly limited, and specific examples thereof include natural fibers (vegetable fibers or animal fibers) such as cotton, silk, fur, wool, and hemp, and synthetic fibers such as acryl, polyester, nylon, vinylon, polyolefin, and polyurethane. Pile is a type of a weaving method and refers to a weaving method for covering the surface of a ground texture of woven fabric with loop-like fibers (loops of thread) protruding longitudinally and laterally from the ground texture for each constant interval. The loops of thread have elasticity. The pile fabrics refer to pile-woven fabrics.

The carriers 2 of this embodiment are formed by bending the rectangular sheet S in an inverted U shape. However, the forms of the carriers 2 may be cylindrical shapes or square tube shapes in which both ends in a longitudinal direction or a width direction are connected to each other. In addition, the number of sheets S constituting the carriers 2 is not limited to one, and two or more sheets may be arranged and provided in parallel.

The sheet S constituting the carriers 2 is bent in an inverted U shape, that is, hung on a horizontal portion at an upper end of a hanger H in a state in which it is folded into two from the center. End portions of the carriers 2 can be installed by being fixed to and suspended on the hanger H using a fastener such as a clip or a hook. The width of a horizontal portion of the upper end of the hanger H in a horizontal direction defines a separation distance between culture surfaces (inner surfaces facing each other) of a pair of carriers 2 constituted of one sheet S.

The hanger H is a member on which the carriers 2 are suspended at a desired height (for example, about 1 m) from its lower end and includes: a rod-like fixing member 10 disposed in a horizontal direction for hanging or fixing the carriers 2 thereon; and a leg member 11 for supporting both ends of the fixing member 10. The hanger H may have a plurality of fixing members 10 in parallel.

The carriers 2 may be installed through, for example, a method of attaching the carriers to a support member such as a rigid frame to make the carriers self-stand or a method of directly providing a fastener or the like below a culture solution supply unit 3 to be described below to suspend the carriers on the fastener in addition to the above-described method.

In the case of directly providing a fastener or the like below the culture solution supply unit 3 to be described below to suspend the carriers 2 on the fastener, a culture solution flows on the carriers through the fastener. Therefore, it is possible to use the fastener as a flow path for supplying the culture solution to the carriers 2 and to reliably install the carriers 2 immediately below the culture solution supply unit 3. Accordingly, it is unnecessary to position the culture solution supply unit 3 and the hanger.

The culture solution supply unit 3 of this embodiment is a horizontally disposed tubular member for supplying a culture solution to the carriers 2 by releasing the culture solution. A part thereof is connected to a culture solution storage tank and a nutrient supply tank which are not shown in the drawing through the circulation flow path 7. A plurality of supply holes 3a for releasing a culture solution are formed on the peripheral wall of the culture solution supply unit 3 at a center portion opposite to upper ends of the carriers 2 at a constant interval in an axial direction. The supply holes 3a are arranged downward, and a culture solution is supplied to the upper ends of the carriers 2 at an approximately uniform water content throughout the whole area of the carriers 2 in a width direction. A plurality of culture solution supply units 3 may be arranged depending on the number of carriers 2 or the arrangement of the carriers 2.

As the capacity of the culture solution supply unit 3 to supply a culture solution, it is desirable to adjust the flow-down rate of the culture solution in the carriers 2 to a range of 5 mL/h/m² to 30,000 mL/h/m² using a control device to be described below. The fluctuation range depends on the proliferation of microorganisms. For example, in the case of *Chlorella,* one cell grows and divides into four, and each grows to its size before the division within 16 hours. Although a small amount of nutrients is sufficient at the beginning of the division, it is necessary to provide enough nutrients throughout the growth phase. Accordingly, it is possible to allow microorganisms to naturally flow down together with the culture solution in a continuous manner while maintaining proliferation by filling the surroundings of the microorganisms with a fresh culture solution at all times. In addition, in a case where a surface layer portion of a microorganism layer attached to the carriers 2 is, if necessary, allowed to forcibly fall by changing the flow rate of the culture solution or applying an impact such as vibration to the carriers 2, photosynthesis at a lower layer portion becomes active and proliferation is performed, thereby increasing the amount of microorganisms collected.

In a case where, for example, the carriers 2 consist of a sheet body which is not a pile fabric of 0.5 m² or more, it is necessary to make a culture solution flow at a flow rate of higher than or equal to 1,000 mL/h/m² at the beginning and then at a flow rate of 5,000 mL/h/m² while gradually increasing the flow rate depending on the planting amount in order to maintain stable cell proliferation of microorganisms such as microalgae and provide minimum moisture and/or nutrients required for facilitating gas (CO₂) exchange. For this reason, the outflow amount of microorganisms increases with an increase in the flow rate of the culture solution up to 1,500 mL/h/m², but the increase in the outflow amount slows down at a flow rate of higher than or equal to 6,000 mL/h/m². The flow rate of the culture solution is preferably higher than or equal to 1,500 mL/h/m². The flow rate of the culture solution can be calculated through the following method. The amount of culture solution flowing out of carriers is measured for 10 seconds during culture. This operation is repeated three times, and an average value (mL/h) of the amount of culture solution per hour is calculated. The flow rate (mL/h/m²) of the culture solution can be calculated by dividing the value by the area of surfaces of the carriers.

In a case where the flow rate is too high, problems may arise in that microorganisms such as microalgae are not easily fixed to the carriers 2, the proliferation rate decreases, it is difficult to perform CO₂ exchange due to a thickened nutrient solution phase, or stress is applied to microorganisms such as microalgae due to physical stimulation.

In a case where the carriers 2 consist of pile fabrics of twisted yarn or non-twisted yarn of 0.5 m² or more, the flow rate of a culture solution flowing on the surfaces of the carriers 2 exceeds 1,200 mL/h/m², is preferably higher than or equal to 5,400 mL/h/m², and is more preferably higher than or equal to 9,000 mL/h/m². The upper limit of the flow rate is preferably lower than or equal to 30,000 mL/h/m², more preferably lower than or equal to 27,000 mL/h/m², and still more preferably lower than or equal to 24,000 mL/h/m².

The culture solution is not particularly limited as long as it is a diluted solution of a medium with which it is possible to increase the concentration of microorganisms by culturing the microorganisms through a usual method. General inorganic media such as a CHU medium, a JM medium, and an MDM medium can be used as the medium, for example. Furthermore, diluted solutions of various media such as a Gamborg's B5 medium, a BG11 medium, and an HSM medium are preferable as the medium. The inorganic medium contains Ca(NO₃)₂·4H₂O, KNO₃ or NH₄Cl as a nitrogen source and KH₂PO₄, MgSO₄·7H₂O, FeSO₄·7H₂O, or the like as other main nutritional components. Antibiotics or the like which do not affect growth of microorganisms may be added to a medium. The pH of a medium is preferably 4 to 10. If possible, waste water or the like discharged from various industries may be used.

The light irradiation unit 4 of this embodiment is a linear device in which LED bulbs (or LEDs) are arranged in a row, lenses that provide an appropriate irradiation angle so as to supply an approximately even amount of light to the surfaces of the carriers 2 to be irradiated with light are respectively arranged opposite to the LED bulbs, and the LED bulbs and the lenses are fixed to rod-like supports. The light irradiation unit appropriately irradiates almost the whole area of the surfaces of the opposite carriers 2 with light having a wavelength or a light amount suitable for proliferation.

The wavelength of light emitted by the light irradiation unit 4 may be, for example, within a range of 380 to 780 nm. The light irradiation unit 4 may irradiate microorganisms such as microalgae, which can proliferate only with red light, only with red light suitable for photosynthesis. Microalgae such as *Chlorella* can favorably proliferate only with red light. Light emitted by the light irradiation unit 4 may be continuously emitted, or may be intermittently emitted light at 100 to 10,000 Hz.

The light irradiation unit 4 is, as shown in FIG. 2, disposed between surfaces of two carriers 2, 2 directly opposed to each other and on an outside of the carriers 2 in a width direction (that is, a horizontal direction) when the carriers 2 are viewed from the side, that is, when viewed in a direction orthogonal to an arrangement direction (a direction of an arrow L) of the carriers 2. It is preferable that the distances between the light irradiation unit 4 and each of a pair of carriers 2 adjacent to each other be substantially equal to each other. The light irradiation unit 4 may be installed on an outside of the carriers 2 in the width direction, that is, at a position as close as possible to side edges of the carriers 2 in parallel to the side edges so as not to overlap the side edges of the carriers 2 when viewed in the arrangement direction (the direction of the arrow L) of the plurality of carriers 2. In the case where the light irradiation unit 4 is positioned further on the outside of the side edges of the carriers 2 when viewed in the arrangement direction of the carriers 2, it is possible to improve the work efficiency when collecting microorganisms from the carriers 2. In addition, in the case where the light irradiation unit 4 is positioned as close as possible to the side edges of the carriers 2, it is possible to improve the uniformity of the amount of light applied to the carriers 2.

The effluent tank 5 is a storage tank of a culture solution containing microorganisms flowing out of the carriers 2 and has a shape of a box having a certain depth and an open upper end so as to receive the culture solution flowing down from the carriers 2. In the effluent tank 5, the culture solution containing microorganisms flowing out of the carriers 2 is separated by gravity into precipitates containing microorganisms at a high concentration and a culture solution which is a supernatant containing almost no microorganisms.

The harvest container 6 is a container that collects and accommodates precipitates, which are separated in the effluent tank 5 and contain microorganisms at a high concentration, by opening a valve 6A from the bottom of the effluent tank 5.

The circulation flow path 7 is for collecting a culture solution (supernatant solution) separated in the effluent tank 5 and supplying the collected culture solution to the carriers 2 again. A pump P is provided on the circulation flow path 7, and the collected culture solution is pumped up above the carriers 2 using the pump. The pumped-up culture solution is continuously supplied from above the carriers 2 again. The culture solution supplied to the carriers 2 again is a supernatant separated in the effluent tank 5, but may contain microorganisms. A strainer may be provided in front of the pump P to strain and collect at least some microorganisms contained in the supernatant solution with the strainer. The pump P is connected to the control device that is not shown in the drawing, and the flow rate is manually controlled or automatically controlled with a predetermined program.

The case 8 of this embodiment has a box shape and covers the entirety of the carriers 2, the culture solution supply unit 3, the effluent tank 5, and the circulation flow path 7. In the case where the carriers 2 are covered with the case 8, the heat insulation capacity further increases and the temperature of the surfaces of the carriers 2 is easily kept constant.

The material of the case 8 is not particularly limited, and examples thereof include transparent materials such as glass, acryl, polystyrene, and vinyl chloride. In a case of culturing microorganisms that can proliferate using a culture system 1 without performing photosynthesis, it is unnecessary for the material of the case 8 to be transparent.

The case 8 is filled with mixed air containing about 1% to 40% CO₂, and it is preferable that CO₂ can be fed so as to be appropriately supplemented thereto. In a case where mixed air contains about 1% to 10% CO₂, it is possible to allow many microorganisms such as microalgae to favorably photosynthesize. Even in a case where atmospheric air is ventilated, microorganisms can proliferate even if the proliferation rate becomes slow.

### [Culture Subject]

Microorganisms which are culture subjects in the culture system of the present invention are not particularly limited and include not only photosynthetic microorganisms, such as *Chlorella, Synechocystis,* and *Spirulina,* which have no or poor mobility, but also planktonic *Euglena* or *Chlamydomonas* and *Pleurochrysis* which have flagella and move in water. The types of microorganisms which are culture subjects in the culture system 1 are extremely diverse. Examples of main microorganism groups which are culture subjects in the culture system 1 include the following groups A to C.

Examples of the group A include eubacteria and archaebacteria which are prokaryotes.

Examples of the eubacteria include non-oxygen-generating photosynthetic bacteria, cyanobacteria performing oxygen-generating photosynthesis, facultative, anaerobic, fermentative bacteria and non-fermentative bacteria which use organic substances, lithotrophic bacteria, actinomycetes, *Corynebacterium,* and spore-bearing bacteria. Examples of the photosynthetic bacteria include *Rhodobacter, Rhodospirillum, Chlorobium,* and *Chloroflexus.* Examples of the cyanobacteria include *Synechococcus, Synechocystis, Spirulina, Arthrospira, Nostoc, Anabaena, Oscillatoria, Lyngbya, Nostoc commune,* and *Aphanothece sacrum.*

Examples of the facultative, anaerobic, fermentative bacteria include *Escherichia coli* and lactic acid bacteria. Examples of the non-fermentative bacteria include *Pseudomonas.* Examples of the lithotrophic bacteria include hydrogen-oxidizing bacteria. Examples of the actinomycetes include *Streptomyces,* and examples of the spore-bearing bacteria include *Bacillus subtilis.* Examples of the archaebacteria include thermophiles or extreme halophiles. Examples of the thermophiles include *Thermococcus,* and examples of the extreme halophiles include *Halobacterium.* Other examples of the group A include glutamic-acid-producing bacteria, lysine-producing bacteria, and cellulose-producing bacteria.

Examples of the group B include microalgae which are eukaryotic, photosynthetic microorganisms.

Examples of the microalgae include green algae, *Trebouxia* algae, red algae, diatoms, haptophyte algae, eustigmatophyte, *Euglena,* and zooxanthellae.

Examples of the green algae include *Chlorella, Scenedesmus, Chlamydomonas, Botryococcus, Haematococcus, Nannochloris,* and *Pseudochoricystis,* and examples of the *Trebouxia* algae include *Parachlorella* or *Coccomyxa.* Examples of the red algae include *Cyanidioschyzon, Cyanidium, Galdieria,* and *Porphyridium,* and examples of the diatoms include *Nitzschia, Phaeodactylum, Chaetoceros, Thalassiosira, Skeletonema,* and *Fistulifera.* Examples of the haptophyte algae include *Pleurochrysis, Gephyrocapsa, Emiliania, Isochrysis,* and *Pavlova.* Examples of *Nannochloropsis oculata* include *Nannochloropsis,* examples of *Euglena* include *Euglena,* and examples of *Prasinophyceae* include *Tetraselmis.* Furthermore, examples of the zooxanthellae as symbiotic algae of coral include *Symbiodinium.*

Examples of the group C include fungi which are non-photosynthetic eukaryotes. Examples of the fungi include yeast and *Aspergillus.* In addition, mycelia of basidiomycetes can be culture subjects.

Ulva or green laver which are green algae, *Pyropia tenera, Porphyra, Pyropia yezoensis,* and *Collema* which are red algae, and other kinds of edible laver among multicellular marine algae are also culture subjects even though these are not microorganisms. Furthermore, mosses which are green plants can also be culture subjects. In addition, lichens which are symbionts can also be culture subjects. Microalgae include cyanobacteria. It is possible to culture oomycetes which do not photosynthesize, such as *Aurantiochytrium,* with an organic waste liquid using the culture system of the present invention, for example.

In the present invention, microorganisms which are culture subjects are preferably photosynthetic microorganisms. In this case, the light irradiation unit 4 is essential for the culture system 1. However, in the case of culturing microorganisms that can proliferate using the culture system 1 without performing photosynthesis, the light irradiation unit 4 may not be used.

Next, a method of using the culture system 1 and its action will be described.

In order to start use of the culture system 1, microorganisms are attached to absorbent cotton or the like which has been placed on the carriers 2, and end portions of the carriers are hung or suspended on the hanger H or the like to be fixed. As the microorganism attachment method, water containing microorganisms may be directly added dropwise or applied to the carriers 2. Air containing about 1% to 40% CO₂ is fed into the case 8 upward from below.

Then, while a culture solution is continuously supplied from the culture solution supply unit 3 so as to flow down in the carriers 2 at a rate of 5 mL/h/m² or higher, red light and/or white light having a wavelength of 380 to 780 nm is emitted by the light irradiation units 4. The light amount (photon flux density) of this light irradiation is set to be low at about 50 µmolm⁻²s⁻¹ at the beginning of planting of microorganisms and increases to about 400 µmolm⁻²s⁻¹ according to growth of the microorganisms. In addition, it is preferable to set a light-off time at an initial stage of the proliferation because of characteristics of photosynthetic organisms dividing at night. At this time, the liquid temperature and the atmospheric temperature of the surfaces of the carriers are preferably set to 33°C to 37°C.

After a certain period of time, the culture solution spreads over the carriers 2, and the culture solution is further supplied from the culture solution supply unit 3. Therefore, the culture solution flows down to the effluent tank 5 from lower ends of the carriers 2. At this time, microorganisms which have been attached to the carriers 2 or cultured in the carriers 2 gradually flow out of the carriers 2 due to the flow of the culture solution and flow down to the effluent tank 5 together with the culture solution.

The microorganisms flowing down from the carriers 2 precipitate in the culture solution of the effluent tank 5 and are introduced into the harvest container 6 by opening the valve 6A installed below the effluent tank 5.

On the other hand, a supernatant solution of the culture solution containing some microorganisms accumulated in the effluent tank 5 is pumped up by the pump P, re-supplied to the culture solution supply unit 3 via the circulation flow path 7, and repeatedly supplied onto the carriers 2.

The amount of new culture solution to be supplied from the culture solution storage tank that is not shown in the drawing is adjusted according to the amount of culture solution which contains microorganisms and is to be re-supplied to the culture solution supply unit 3, and necessary nutrients are appropriately supplied to the culture solution supply unit 3 from the nutrient supply tank that is not shown in the drawing and released to the carriers 2 together with the culture solution.

Some microorganisms naturally flow down from the carriers 2 as described above. In this embodiment, surface layers of microorganism layers fixed on the carriers 2 may be scraped off depending on division and growth of the microorganisms. Accordingly, photosynthesis of microorganisms at a lower layer portion is also activated, and division and proliferation start. By repeating the above-described operation, cultured microorganisms are harvested while the microorganisms are continuously cultured.

According to the culture system 1 of the present embodiment, it is possible to reduce the installation intervals among a plurality of carriers 2 to be arranged. Therefore, it is possible to increase the installation density of the plurality of carriers 2 and light irradiation units 4 with respect to the floor area on which the microorganism culture system 1 is installed.

In addition, in the microorganism culture system 1, the rod-like light irradiation units 4 are arranged between the carriers 2 disposed opposite to each other and on both outer sides of the carriers 2 in a width direction. Therefore, even in a case of raking out microorganisms cultured on the surfaces of the carriers 2, the microorganisms can be easily collected without being obstructed by the light irradiation units 4.

In addition, it is possible to apply a sufficient amount of light to microorganisms attached to the culture surfaces of the carriers 2 simply by arranging the light irradiation units 4 on side portions of the carriers 2. Therefore, it is possible to culture the microorganisms with high energy efficiency.

In addition, in the microorganism culture system 1, the light irradiation units 4 are used in which a plurality of LED bulbs are arranged in a row and lenses that can perform adjustment so that an even amount of light is supplied to the entire surfaces of the carriers 2 regardless of the size of the surfaces of the carriers 2 are respectively arranged in front of the LED bulbs. Accordingly, in the microorganism culture system 1, it is possible to reliably culture the microorganisms by providing a sufficient amount of light even if light is emitted from an oblique direction of the carriers 2.

In the above-described embodiment, an example in which a light irradiation unit 4 is installed to correspond to each inner surface (culture surface) of a sheet S constituting a pair of carriers 2 directly opposed to each other is illustrated as shown in FIG. 3. However, microorganisms can be cultured even on surfaces facing outsides of the carriers 2. Therefore, the light irradiation unit 4 may be further arranged even between carriers 2 which are hung on adjacent separate sheets S as shown by a virtual line in FIG. 1.

In addition, in a case where there are three or more carriers 2 arranged, the light irradiation units 4 and the carriers 2 can be alternately arranged as shown in FIG. 3.

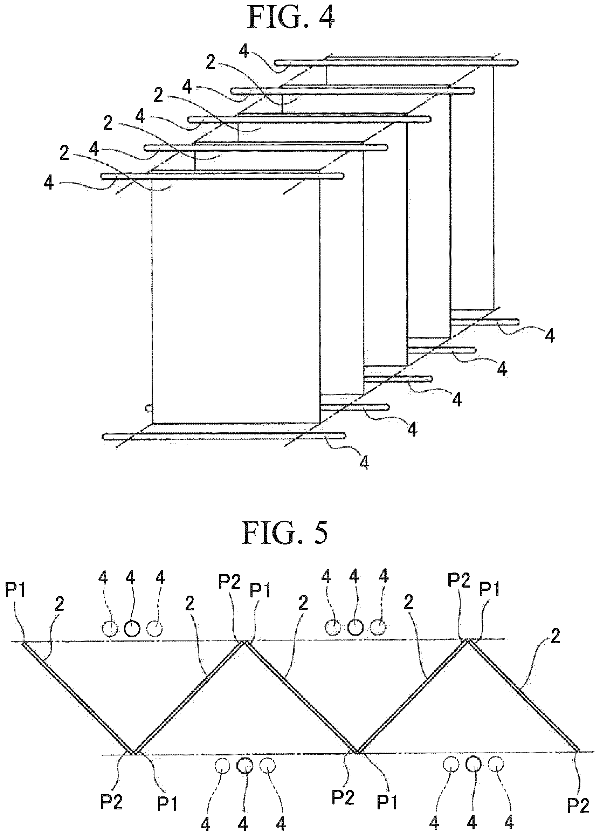

In the case where there are three or more carriers 2 arranged, the light irradiation units 4 may be arranged on either one or both of outsides of the carriers 2 in the vertical direction as shown in FIG. 4. An aspect in which the aspect shown in FIG. 3 is combined with the aspect shown in FIG. 4 may be employed for the light irradiation units 4. That is, the light irradiation units 4 may be arranged on either one or both of outsides of left and right edges of the carriers 2 and on either one or both of outsides of upper and lower edges thereof.

In the above-described embodiment and the modification example thereof, an example in which the carriers 2 are arranged so that surfaces of adjacent carriers 2 are all parallel to each other, that is, the plurality of carriers 2 are arranged so as to be directly opposed to each other, is shown. However, the carriers 2 may not be directly opposed to each other.

For example, in the embodiment shown in FIG. 5, surfaces of adjacent carriers 2 are not parallel to each other, but are arranged in an L shape or a zigzag shape at a certain angle when viewed in plan view. In the embodiment shown in FIG. 5, the adjacent carriers 2 are arranged so as to form an angle of about 90° when viewed in plan view. Side end portions P1 and P2 of the adjacent carriers 2 are arranged at small intervals or in contact with each other.

Light irradiation units 4 are provided at positions facing valley portions of the L shape formed by the side end portions P1 and P2 of the adjacent carriers 2. That is, the light irradiation units 4 are provided near the outside at a position where a bisector at a corner on a valley side intersects with a virtual plane (shown by an alternate long and short dash line in FIG. 5) connecting left and right edges of the carriers 2 when viewed in the arrangement direction of the carriers 2. In addition to or instead of the position of FIG. 5, the light irradiation units 4 may be provided on the outside at a position where a bisector at a corner on a valley side intersects with a virtual plane connecting upper and lower edges of the carriers 2 when viewed in the arrangement direction of the carriers 2.

In the case where the plurality of carriers 2 and light irradiation units 4 are arranged in this manner, the above-described action, function, and effect are exhibited. In addition, there are effects that it is possible to effectively emit light by causing the light irradiation units 4 to be directly opposed to the surfaces of the carriers 2 and to improve the culture efficiency of microorganisms. The expression that "the light irradiation units 4 are caused to be directly opposed to the surfaces of the carriers 2" means that the light irradiation units 4 are arranged so that at least some light of the light irradiation units 4 is perpendicularly applied to the surfaces of the carriers 2. As shown by the virtual line in FIG. 5, the plurality of light irradiation units 4 may be arranged at the above-described positions.

Regarding other constituent elements, the present invention is not limited to the configuration of the embodiment. For example, collection of microorganisms from a culture solution stored in the effluent tank 5 may be performed by any one of filtration, centrifugal processing, or natural precipitation. In the case of harvesting substances which microorganisms discharge outside cells, other methods such as adsorption or concentration are applied.

The carriers 2 may be covered with a sheet capable of keeping the carriers 2 moderately warm together with the culture solution supply unit 3. In this case, a translucent sheet-like member made of a synthetic resin such as vinyl, polyethylene, or polyester is suitably used as the sheet body.

In the embodiment, vertically long carriers are provided, but horizontally long carriers may be used. In addition, some or all of the above-described various configurations may be appropriately combined as long as the configurations do not depart from the gist of the present invention.

### EXAMPLES

The present invention will be described below in more detail with reference to examples, but the present invention is not limited to these examples.

### [Water Retention Test]

In the present specification, the "water capacity" of a carrier was measured through the following method.
[1] A sample for measuring a water capacity was prepared in a size of 3 cm x 26 cm, and the dry weight was measured.
[2] The sample was placed in a container filled with a sufficient amount of tap water at room temperature (for example, 23°C) and allowed to stand for 3 minutes to sufficiently incorporate the water into the sample.
[3] One end of the sample in a longitudinal direction was pinched with forceps, and the sample was taken out of the container while being stretched in a vertical direction and allowed to stand for 5 seconds in a state in which it was lifted from the water surface to wait for the water to drip off.
[4] The weight of the sample containing water was measured. Even if water dripped at this point in time, the weight including the water that dripped was measured.
[5] The dry weight of the sample was subtracted from the weight measured in [4], and the amount of water contained per 1 cm² of the sample was calculated.

The measurement was performed 5 times for each sample, and the average value was regarded as a "water capacity (g/cm²)".

### [Example 1]

*Chlorella* (*Chlorella kessleri* 11h) was cultured using the culture system 1 shown in FIG. 1.

Carriers obtained by folding a pile fabric sheet (water capacity: 0.395 g/cm²) woven with non-twisted yarn of 50 cm wide x 120 cm long in two, placing the sides of the folded sheet directly opposite to each other, and suspending the sheet on the member 10 of the hanger H in an inverted U shape were used as the carriers 2. A pump P (trade name "1046" manufactured by EHEIM) was provided on a downstream side of the circulation flow path 7. A commercially available glass case (the thickness of the glass being 3 mm) was used as the case 8. A red line type LED module (manufactured by Effect) was used as the light irradiation unit 4.

Air containing 10 volume% CO₂ was introduced into the case 8 upward from below at a rate of about 1.0 L/min using the culture system 1. The air was allowed to flow downward from above, and at the same time, was bubbled into a medium in the effluent tank 5. A solution obtained by adding KNO₃ to a dilution obtained by diluting a plant tissue culture medium Gamborg B5 by a factor of 50 so that it had a concentration of 150 mg/L was used as a culture solution. *Chlorella* was cultured at 33°C to 37°C while the culture solution was supplied at a rate of 1,000 mL/h and red light having an intensity of 50 µmolm⁻²s⁻¹ was emitted from a horizontal direction as shown in FIG. 3. At the start of the culture, 15 g of *Chlorella* by dry weight was attached to absorbent cotton placed on the carriers 2 to start the culture.

The concentration of the medium was adjusted so that the Gamborg's B5 medium was diluted by a factor of 10 2 hours after the start of the culture. From the next day, the medium of the effluent tank 5 was replaced once a day with a culture solution obtained by adding 750 mg of KNO₃, 5 ml of a nutritional supplement (which did not contain glucose, but contained 17 g/l of NaH₂PO₄, 15 g/l of MgSO₄, and 13 g/l of (NH₄)₂SO₄ as the composition for returning to the 10-fold dilution of the Gamborg's B5 medium), and 50 µl of NH₄CL to a 10-fold dilution of the Gamborg's B5 medium per liter of the medium. The amount of light was set to 100 µmolm⁻²s⁻¹ from the next day after the start of the culture. The surface temperature of the carriers 2 was constantly 33°C to 35°C during the culture.

The culture solution containing *Chlorella* flowing out of the carriers 2 was collected in the effluent tank 5. The effluent tank 5 was covered with a black cloth in order to prevent proliferation of *Chlorella* in the effluent tank 5. Collection of *Chlorella* was performed by scraping the surfaces of the carriers 2 once to three times a day from day 3 after the start of the culture and subjecting the culture solution in the effluent tank 5 to centrifugal processing. The collected *Chlorella* was suspended in the culture solution again, and the dry weight (0.35 of the turbidity at 730 nm = 1 g DW (dry weight)/L) was calculated from the turbidity at 730 nm measured with a spectrophotometer (DU700 manufactured by Beckman Coulter). In addition, the dry weight was obtained and confirmed also from *Chlorella* dried for 2 hours or longer at 80°C. As a result of the culture, it was possible to harvest *Chlorella* with a dry weight of 169.56 g/m² (which was calculated per m² of a carrier 2) on day 5 after the start of the culture.

### [Example 2]

*Chlorella* was cultured in the same manner as in Example 1 except that pile fabrics (water capacity: 0.267 g/cm²) woven with twisted yarn of 50 cm wide x 120 cm long were used as the carriers 2 and light was emitted from the lateral and horizontal directions in FIGS. 3 and 4 from light sources, and the dry weight was calculated. As a result of the culture, it was possible to harvest *Chlorella* having a dry weight of 157.07 g/m² on day 5 of the culture.

### [Industrial Applicability]

The microorganism culture system of the present invention is industrially applicable because it can improve the efficiency of installing light sources and carriers with respect to a floor area, allows microorganisms to be easily collected from the carriers, and allows microorganisms to be efficiently produced using light irradiation units with lower energy consumption.

### [Reference Signs List]

- 1: microorganism culture system
- 2: carrier
- S: sheet
- H: hanger
- 3: culture solution supply unit
- 3a: supply hole
- 4: light irradiation unit
- 5: effluent tank
- 6: harvest container
- 7: circulation flow path
- P: pump
- 8: case

## Claims

1. A microorganism culture system, comprising:
flat carriers to which microorganisms are to be attached;
a culture solution supply unit that supplies a culture solution to the carriers from above the carriers; and
an effluent tank that stores a culture solution containing the microorganisms flowing out of the carriers,
wherein the plurality of carriers are arranged so that surfaces of the carriers are directly opposed to each other or obliquely face each other at an angle, and
wherein light irradiation units are installed between the plurality of arranged carriers and in at least a part of an outside of the carriers in a horizontal direction and an outside thereof in a vertical direction when viewed in an arrangement direction of the carriers.

2. The microorganism culture system according to Claim 1,
wherein the light irradiation units are arranged so as to be directly opposed to the surfaces of the carriers.

3. The microorganism culture system according to Claim 1 or 2,
wherein a photon flux density on the surfaces of the carriers in a wavelength range in which the microorganisms can be absorbed is greater than or equal to 50 µmοlm⁻²s⁻¹.

4. The microorganism culture system according to any one of Claims 1 to 3,
wherein a plurality of LED bulbs are arranged in a row as the light irradiation units, and lenses that can perform adjustment so that an even amount of light is supplied to the entire surfaces of the carriers are arranged opposite to each of the bulbs.

5. The microorganism culture system according to any one of Claims 1 to 4,
wherein the light irradiation units are arranged between side edges that face each other in the arrangement direction of two adjacent carriers.

6. The microorganism culture system according to any one of Claims 1 to 5,
wherein the microorganisms are microalgae.

7. The microorganism culture system according to any one of Claims 1 to 6,
wherein a pair of the flat carriers are formed by bending a sheet in an inverted U shape and hanging the sheet, and the light irradiation units are installed between both side edges, which face each other, of the carriers and in at least a part of an outside of the carriers in a horizontal direction and an outside thereof in a vertical direction when viewed in the arrangement direction of the carriers.

8. The microorganism culture system according to any one of Claims 1 to 6,
wherein each of the carriers has a rectangular shape extending in the vertical direction, a plurality of the carriers are arranged so as to form a zigzag shape in plan view, and the light irradiation units are arranged at positions facing valley portions of the zigzag and further on an outside of a virtual plane connecting crest portions of the zigzag.
